# EUROPÄISCHE PATENTANMELDUNG

(11) **EP 2 786 734 A2**
(43) Veröffentlichungstag der Anmeldung: **08.10.2014**
(21) Anmeldenummer: 14001127.1
(22) Anmeldetag: 26.03.2014
(51) Int. Cl.: A61K 6/00, A61K 6/06

(54) **Feinkörniges Dentalmaterial**

(30) Priorität: 03.04.2013 DE 102013005689
(71) Anmelder: Kiefl, Matthias, 94315 Straubing (DE); Kozluklu, Savas, 85253 Erdweg (DE)
(72) Erfinder: Kiefl, Matthias, 94315 Straubing (DE); Kozluklu, Savas, 85253 Erdweg (DE)
(74) Vertreter: Samson & Partner

(57) **Zusammenfassung**

Ein Dentalmaterial umfasst einen Zementklinker, der aus Portlandzementklinker oder einem Portlandzement ähnlichen gipsfreien Zementklinker ausgewählt ist und bei dem 90 Volumen-% der Teilchen des Zementklinkers eine Größe von etwa 6 µm oder weniger aufweisen. Die Verwendung und die Herstellung des Dentalmaterials werden ebenfalls beschrieben.

## Beschreibung

### Gebiet der Erfindung

Die Erfindung betrifft ein Dentalmaterial, das einen Zementklinker umfasst, der aus Portlandzementklinker oder einem Portlandzementklinker ähnlichen Zementklinker ausgewählt ist, sowie die Verwendung des Dentalmaterials und dessen Herstellung.

### Hintergrund der Erfindung

Die Verwendung von Zement und Wasser zum Versiegeln oder Füllen von Zahnkavitäten wurde erstmals von Mahmoud Torabinejad und Dean J. White in den US-Patenten 5,415,547 und 5,769,638 beschrieben. Die bevorzugten Zemente waren Portlandzemente, insbesondere normaler Portlandzement oder Portlandzement vom Typ I, und wiesen einen Blaine-Wert (eine mit dem Blaine-Gerät gemessene Oberfläche) von 4.000 - 5.500 cm²/g, bevorzugt 4.500 - 4.600 cm²/g auf. Dies entspricht d₉₀-Werten von jedenfalls mehr als 12 µm (d₉₀ ist die maximale Teilchengröße, die 90 Masse-% der Teilchen der Probe aufweisen).

In der US-Patentanmeldungsveröffentlichung US 2005/0263036 A1 wird die Verwendung von weißem Portlandzement, der nur einen sehr geringen Eisenoxid-Anteil aufweist, oder Calciumaluminium-Zement oder deren Mischung in einem Dentalmaterial beschrieben. Die Teilchengröße d₉₀ des weißen Portlandzements mit einer Oberfläche von 409 m²/kg (4.090 cm²/g) liegt bei 25 µm, wobei die Teilchengröße d₅₀, welche die maximale Teilchengröße von 50 Masse-% der Teilchen der Probe ist, als 9 µm angegeben wird. Für Wurzelbehandlungen wird ein feineres Material mit einer Oberfläche bis zu etwa 1.000 cm²/g bevorzugt (dies entspricht einer Teilchengröße mit d₉₀ von jedenfalls größer als 8.6 µm). Das Dentalmaterial kann ferner ein Röntgenkontrastmittel, z.B. Bismutoxid, Bariumsulfat, Bariumoxid (in der Form eines Ersatzes von einem Teil des Calciumoxids des Zements) oder ein röntgenundurchlässiges Glas, Fluorid in Form von Calciumfluorid oder Bioglas enthalten.

Ein Zementmaterial, das einen Zementklinker, der Portland-Zementklinker ähnlich ist (d.h. einen Zementklinker, der vorwiegend aus Tricalciumsilicat ((CaO)₃-SiO₂), Dicalciumsilicat ((CaO)₂-SiO₂), Tricalciumaluminat ((CaO)₃·Al₂O₃) und kleinere oder größere Mengen an Tetracalciumaluminatferrit ((CaO)₄·Al₂O₃·Fe₂O₃) besteht), Gips (CaSO₄·2 H₂O) und Bismutoxid (Bi₂O₅) als Röntgenkontrastmittel umfasst, wird als Mineraltrioxidaggregat bezeichnet und ist in verschiedenen Ausführungsformen u.a. unter den Handelsnamen ProRootMTA^{®}, W-MTA^{®}, G-MTA^{®}, MTA-Angelus^{®}, Medcem MTA^{®}, Harvard MTA^{®}, Cumdente MTA^{®} und Ledermix MTA^{®} auf dem Markt. Der d₉₀ und d₅₀ des Produkts ProRootMTA^{®} wurde auf Veranlassung der gegenwärtigen Erfinder in einem Labor gemessen und beträgt 22,12 µm bzw. 6,186 µm.

Dieses Material weist jedoch Nachteile auf, insbesondere eine geringe Viskosität, schlechte Kohäsion und lange Härtungszeit.

In darauffolgenden Patentanmeldungen wurden Vorschläge insbesondere zur Erhöhung der Viskosität vorgebracht. So schlägt die WO 2005/039509 A1 den Zusatz eines organischen oder anorganischen viskositätserhöhenden Mittels, z.B. Poly(vinylalkohol) oder eine wässrige Tondispersion, zu einem Portlandzement mit einem Blaine-Wert zwischen 4600 und 5500 cm²/g vor. Die US 2007/0009858 A1 empfiehlt den Zusatz von Cellulose und Calciumchlorid (ohne dabei einen Blaine-Wert zu nennen). Die WO 2011/023199 offenbart die Zugabe eines Superplasticizers sowie teilchenförmiger Zusätze mit Oberflächen zwischen 200 und 300.000 m²/kg, zu einer Zementzusammensetzung, die keinen Gips enthält, zur Verbesserung der Viskosität und Härtungszeit. Die Oberfläche des Zements soll im Bereich von 400 bis 600 m²/kg oder auch bei 1000 m²/kg liegen.

Die Erfindung hatte zum Ziel, eine Verbesserung der physikalisch-chemischen und mechanischen Eigenschaften von Dentalmaterialien auf der Basis von

Portlandzementklinker oder einem ähnlichen Zementklinker möglichst ohne chemische Zusätze zu erreichen.

### Zusammenfassung der Erfindung

Die Erfindung betrifft ein Dentalmaterial, umfassend einen Zementklinker, bei dem es sich um Portlandzementklinker oder einen Portlandzementklinker ähnlichen gipsfreien Zementklinker handelt, bei dem etwa 90 Volumen-% der Teilchen des Zementklinkers, wobei Volumen das kumulierte Volumen aller Teilchen ist, eine Größe von etwa 6 µm oder weniger aufweisen.

Ferner betrifft die Erfindung die Verwendung des Dentalmaterials nach einem der Ansprüche 1 bis 11 zum Füllen oder Verschließen von Zahn- und/oder Zahnwurzelkavitäten und zum Abdecken von Pulpa und/oder Parodontium.

Die Erfindung betrifft auch noch ein Verfahren zur Herstellung des Dentalmaterials, welches dadurch gekennzeichnet ist, dass Portlandzementklinker oder ein Portlandzement ähnlicher gipsfreier Zementklinker in einem oder mehreren Mahlvorgängen in einer oder mehreren verschiedenen Mühlen gemahlen wird.

### Detaillierte Beschreibung

Es wurde überraschend gefunden, dass allein die gründliche Zerkleinerung des Zementklinkers, der in einem Dentalmaterial enthalten ist, nicht nur die mechanischen Eigenschaften, sondern auch die physikalisch-chemischen Eigenschaften des Dentalmaterials wesentlich verbessert.

Erfindungsgemäß wurde Portlandzementklinker oder ein Portlandzementklinker ähnlicher gipsfreier Zementklinker auf eine Teilchengröße zerkleinert, bei der 90 Volumen-% der Teilchen, wobei sich Volumen auf das kumulierte Volumen aller Teilchen bezieht, eine Größe von etwa 6 µm oder weniger, bevorzugt etwa 5 µm oder weniger und besonders bevorzugt etwa 4,5 µm oder weniger aufweisen. Außerdem weisen bevorzugt 99 Volumen-% der Teilchen des Portlandzementklinkers oder des einem Portlandzement ähnlichen gipsfreien Zementklinkers eine Größe von etwa 9 µm oder weniger, bevorzugter von etwa 8 µm oder weniger und besonders bevorzugt von etwa 7 µm oder weniger auf. Ferner ist es bevorzugt, dass 50 Volumen-% der Teilchen des Zementklinkers eine Größe von etwa 4 µm oder weniger, bevorzugter etwa 3 µm oder weniger und besonders bevorzugt von etwa 2 µm oder weniger aufweisen.

Besonders bevorzugt ist demnach ein Dentalmaterial, das Portlandzementklinker oder einen Portlandzement ähnlichen gipsfreien Zementklinker enthält, bei dem 90 Volumen-% der Teilchen ein Größe von etwa 4,5 µm oder weniger aufweisen, 99 Volumen-% der Teilchen ein Größe von etwa 7 µm oder weniger aufweisen und 50 Volumen-% der Teilchen ein Größe von etwa 2 µm oder weniger aufweisen, wie durch Laserbeugung bestimmt.

Zementklinker, z.B. Portlandzementklinker, ist eine durch Brennen von dem Fachmann bekannten Materialien hergestellte Vorstufe von Zement, z.B. Portlandzement, die keine Gipsbeimischung enthält. Durch Mischen mit zumindest Gips wird dann der fertige Zement hergestellt, der insbesondere im Baugewerbe verwendet wird.

Der in dem erfindungsgemäßen Dentalmaterial enthaltene Zementklinker kann aus Portlandzementklinker gemäß der Norm EN 197-1:2000, Kapitel 5.2.1, oder Portlandzement ähnlichem gipsfreiem Zementklinker ausgewählt sein, der Tricalciumsulfat (Alit, CeS), Dicalciumsulfat (Belit, C2S), Tricalciumaluminat (C3A) und gegebenenfalls Tetracalciumaluminatferrit (C4(A,F) und C2(A,F)) als seine Hauptbestandteile (d.h. insgesamt zu mehr als etwa 93 Masse-%) enthält und ohne Gipszusatz ist. MgO ist bevorzugt zu weniger als etwa 2 Masse-%, mehr bevorzugt zu höchstens 1 Masse-% in dem Zementklinker enthalten. Ferner enthält der Zementklinker Schwermetalle außer Eisen bevorzugt nicht oder nur in Spuren (Zn, Ni und Mn < 3·10⁻² Masse-%, Hg < 1,5·10⁻⁵ Masse-%, andere < 3·10⁻³ Masse-%). Es ist weiter bevorzugt, dass SO₃, Cl und Alkali jeweils in Mengen unter etwa 1 Masse-% im Zementklinker vorliegen.

Bei dem in dem erfindungsgemäßen Dentalmaterial enthaltene Zementklinker kann es sich um grauen Portlandzementklinker handeln, der Tetracalciumaluminatferrit in Mengen von mehr als etwa 2 Masse-% und gewöhnlich bis etwa 5 oder 6 oder auch 8 Masse-% enthält. Es kann sich aber auch, und dies bevorzugt, um weißen Portlandzementklinker oder Weißzementklinker handeln, der Tetracalciumaluminatferrit in Mengen von weniger als 2 Masse-%, bevorzugt von etwa 1,1 Masse-% oder weniger, enthält.

Ein besonders bevorzugter Zementklinker ist ein Weißzementklinker, dessen Hauptbestandteile etwa etwa 74 bis etwa 76, z.B. etwa 75 Masse-% Tricalciumsulfat, etwa etwa 14 bis etwa 16, z.B. etwa 15 Masse-% Dicalciumsulfat, weniger als etwa 5 Masse-%Tricalciumaluminat und etwa 0,9-1,1, z.B. etwa 1 Masse-% Tetracalciumaluminatferrit sind.

Für die Zwecke der Sichtbarmachung im Röntgenbild enthält das erfindungsgemäße Dentalmaterial bevorzugt ein Röntgenkontrastmittel, das z.B. aus Bismutoxid, Bariumoxid, Zirkoniumdioxid, Bariumsulfat, röntgenundurchlässigem Keramikmaterial und röntgenundurchlässigem Glas ausgewählt sein kann. Bariumsulfat mit einer Teilchengröße, die etwa in demselben Größenbereich wie die Teilchengröße des erfindungsgemäß eingesetzten Zementklinkers oder darunter, z.B. sogar im Nanometerbereich, liegt, ist bevorzugt.

Das Röntgenkontrastmittel liegt in dem Dentalmaterial im Allgemeinen in einem Verhältnis Kontrastmittel: Zementklinker von etwa 3:7 bis etwa 1:9, bevorzugt etwa 2:8 vor.

Das erfindungsgemäße Dentalmaterial kann, falls gewünscht, weitere Zusätze enthalten. Dazu gehören zum Beispiel CaCl₂, das als Abbindebeschleuniger wirken kann, Epoxide, welche die Viskosität des Dentalmaterials, wenn es für die Anwendung am Zahn mit Wasser gemischt wird, noch erhöhen können, Polycarboxylat, das den Wasserbedarf beim Mischen mit Wasser herabsetzen kann, sowie Fluorid freisetzende Zusätze, die für diesen Zweck bekannt sind, z.B. CaF2, CaFPO3 oder Ca₅(PO₄)F.

Es hat sich herausgestellt, dass es für eine hohe Abbindegeschwindigkeit im Zahn vorteilhaft ist, wenn das Dentalmaterial völlig frei von Gips ist, was deshalb bevorzugt ist. Für spezielle Fälle, bei denen eine langsamere Abbindegeschwindigkeit erwünscht ist, kann das Dentalmaterial jedoch auch noch Gips, im Allgemeinen mit einem Anteil von etwa 1 bis etwa 10, bevorzugt bis etwa 6 Masse-%, bezogen auf den Anteil an Zementklinker des Dentalmaterials, enthalten.

Das trockene Dentalmaterial wird zur Einführung in eine Zahnkavität mit Wasser, bevorzugt destilliertem oder entionisiertem Wasser gemischt. Das Mischungsverhältnis richtet sich nach der gewünschten Konsistenz (Viskosität) des einzuführenden Dentalmaterials. Wenn z.B. nur ein ganz kleine Zementmenge zum Verschließen einer winzigen Kavität benötigt wird, kann es vorteilhaft sein, nur einen Tropfen Wasser zu dem erfindungsgemäßen Dentalmaterial zu geben, so dass man ein sehr viskose Paste erhält, die mit einem geeigneten Instrument leicht an ihren Platz gebracht werden kann. Bei einer großflächigeren Anwendung kann eine geringere Viskosität des einzuführenden Dentalmaterials vorteilhaft sein, wobei das trockene Material dann mit einer größeren Menge Wasser als im vorstehend genannten Fall zum Erhalt einer weniger viskosen Paste gemischt wird. Im Allgemeinen beträgt das Massenverhältnis Zementklinker: Wasser im einsatzfertigen Dentalmaterial etwa 1:0,05 bis etwa 1:0,55, kann aber in speziellen Fällen auch außerhalb dieses Bereichs liegen. Dementsprechend wird beispielsweise ein trockenes Dentalmaterial, das etwa 80 Masse-% Zementklinker und etwa 20 Masse-% Bariumsulfat enthält, in vielen Fällen bevorzugt mit etwa 0,30- 0,40, besonders bevorzugt mit etwa 0,35 Masseprozent Wasser, bezogen auf die Masse des trockenen Dentalmaterials, gemischt.

Das mit Wasser gemischte Material hat die Beschaffenheit einer feinen, viskosen Paste mit guter Kohäsion, deren Körner beim Zerreiben in der Hand kaum oder nicht wahrzunehmen sind, vergleichbar einer Aufschlämmung von Puderzucker. Die Viskosität kann derart eingestellt werden, dass die Paste mit beispielsweise Pluggern, Applikatoren, auch speziellen endodontischen Applikatoren und MTA-Applikatoren, MTA-Gun, MTA-Block und MTA-Plugger, auch in schwer zugängliche Stellen, wie Pulpencavum, Wurzelbereich apikal, Wurzelbereich lateral, wurzelnahe Dentinschichten oder in den Wurzelkanal direkt, eingebracht werden kann.

Nach dem Mischen mit Wasser wird das erfindungsgemäße Dentalmaterial sofort in eine Zahnkavität eingeführt. Falls das Material während der Verarbeitung auf der Mischplatte zu schnell aushärtet, kann es mit wenig Wasser wieder in einen pastösen Zustand überführt werden.

Das erfindungsgemäße Dentalmaterial eignet sich als Zahnzement für alle Versiegelungen und Füllungen, beispielsweise für eine direkte Überkappung der offenen Pulpa, eine Karies Profunda-Behandlung in Form einer Unterfüllung (Abdeckung des pulpennahen Dentins), Wurzelfüllungen allgemein vor allem zum apikalen Verschluss des Wurzelcavums, zum Schluss einer Perforation (zum Beispiel Furkations- / Wurzelperforation), eine Abdichtung des Zahnes von innen gegen die umliegenden Gewebe (Parodontium), zum retrograden Verschluss einer Wurzel, zur Abdeckung nach Pulpenamputation oder Pulpotomie, zur Apexifikation, zur Abdeckung oder zum Füllen von internen oder externen Wurzelresorptionen sowie zur anterograden Wurzelfüllung/einzeitigen definitiven Wurzelfüllung.

Die Verarbeitungszeit, d.h. Abbindezeit des erfindungsgemäßen Dentalmaterials bis zu dem Zeitpunkt, wo es so hart ist, dass es nicht mehr verarbeitet werden kann, beträgt je nach Menge des zugegebenen Wassers etwa 5 Minuten bis weniger als etwa 2 Minuten. Ein eventueller Gipszusatz bewirkt eine längere Abbindezeit. Nach dieser Zeit ist das Material in der Zahnkavität so hart, dass, falls erforderlich, eine weitere Behandlung der Zahnkavität, z. B. durch eine Füllung, eine provisorische Füllung, eine Unterfüllung, eine Wurzelfüllung, eine medikamentöse Einlage, ein Inlay oder eine Krone, erfolgen kann. In jedem Fall sollte jedoch ein Kontakt oder eine Abdeckung des erfindungsgemäßen Dentalmaterials mit säurehaltigen Materialien vermieden werden, um den therapeutisch wichtigen hohen pH des Dentalmaterials nicht zu neutralisieren.

Das erfindungsgemäße Zahnfüllmaterial passt sich besonders gut an kleine Unebenheiten auf der Zahnoberfläche an.

Das Zerkleinern des Zementklinkermaterials erfolgt gegebenenfalls unter Durchführung von mehreren Mahlvorgängen in einer oder mehreren geeigneten Mühlen. Mühlen für die Vermahlung von Zementklinker sind dem Fachmann bekannt. Dazu zählen verschiedene Arten von Kugelmühlen, Vertikalmühen, Horizontalmühlen und Rollenpressen. Für kleinere Klinkermengen können auch andere Mühlen eingesetzt werden, z.B. eine Gegenstrahlmühle.

Die folgenden Beispiele erläutern die Erfindung.

### Beispiele

### Beispiel 1

160 g Weißklinker mit einer Aufgabenkörnung von 10,8 % < 2 µm, 24,6% < 5 µm, 47,1 % < 10 µm, 79,3 % 20 µm und 99,97 % < 60 µm wurden in einer Gegenstrahlmühle bei einem Mahldruck von 7 bar gemahlen, bis ein Material erhalten wurde, bei dem 90 Volumenprozent der Teilchen eine Größe bis 4 µm hatten, 50 Volumen-% der Teilchen eine Größe bis 2 µm hatten und 99 Volumen-% der Teilchen eine Größe bis 7µm hatten, wie durch Laserbeugung bestimmt.

### Beispiel 2

800 mg des in Beispiel 1 erhaltenen Materials und 200 mg pulverförmiges Bariumsulfat (mittlere Teilchengröße 3,86 µm, wobei 99 Vol.-% der Teilchen eine Größe bis 9,73 µm aufwiesen) wurden gründlich gemischt und weiter mit 0,35 ml destilliertem Wasser zu einer Paste verrührt, die mit einer ganz feinen Amalgampistole aufgenommen und in die Kavität eines extrahierten Zahnes gespritzt wurde. Die Abbindezeit im Zahn bis zu dem Punkt, wo das Material so hart war, dass es nicht mehr weiter verarbeitet werden konnte, betrug etwa 2 min.

## Patentansprüche

1. Dentalmaterial, umfassend einen Zementklinker, der aus Portlandzementklinker oder einem Portlandzement ähnlichen gipsfreien Zementklinker ausgewählt ist und bei dem 90 Volumen-% der Teilchen des Zementklinkers eine Größe von etwa 6 µm oder weniger aufweisen.

2. Dentalmaterial nach Anspruch 1, **dadurch gekennzeichnet, dass** das Dentalmaterial frei von Gips ist.

3. Dentalmaterial nach Anspruch 1 oder 2, **dadurch gekennzeichnet, dass** 90 Volumen-% der Teilchen des Zementklinkers eine Größe von etwa 5 µm oder weniger, bevorzugt 4,5 µm oder weniger aufweisen.

4. Dentalmaterial nach einem der Ansprüche 1 bis 3, **dadurch gekennzeichnet, dass** 50 Volumen-% der Teilchen eine Größe von etwa 4 µm oder weniger, bevorzugt etwa 3 µm oder weniger und insbesondere etwa 2 µm oder weniger aufweisen.

5. Dentalmaterial nach einem der Ansprüche 1 bis 4, **dadurch gekennzeichnet, dass** 99 Volumen-% der Teilchen eine Größe von etwa 9 µm oder weniger, bevorzugt etwa 8 µm oder weniger und insbesondere etwa 7 µm oder weniger aufweisen.

6. Dentalmaterial nach einem der Ansprüche 1 bis 5, **dadurch gekennzeichnet, dass** der Zementklinker einen MgO-Gehalt von weniger als 2 Masse-% aufweist.

7. Dentalmaterial nach einem der Anspruch 1 bis 6, **dadurch gekennzeichnet, dass** der Zementklinker ein grauer Portlandzementklinker ist.

8. Dentalmaterial nach einem der Ansprüche 1 bis 6, **dadurch gekennzeichnet, dass** der Zementklinker ein weißer Portlandzementklinker ist, der die folgende Zusammensetzung umfasst:
- Tricalciumsulfat: etwa 74-76 Masse-%
- Dicalciumsulfat: etwa 14-16 Masse-%
- Tricalciumaluminat: < etwa 5 Masse-%
- Tetracalciumaluminatferrit: etwa 0,9 - 1,1 Masse-%.

9. Dentalmaterial nach einem der Anspruch 1 bis 8, **dadurch gekennzeichnet, dass** es ferner ein Röntgenkontrastmittel umfasst.

10. Dentalmaterial nach Anspruch 9, **dadurch gekennzeichnet, dass** das Röntgenkontrastmittel Bariumsulfat umfasst.

11. Dentalmaterial nach einem der Anspruch 1 bis 10, **dadurch gekennzeichnet, dass** es ferner Wasser, bevorzugt in einem Massenverhältnis Zementklinker: Wasser von etwa 1:0,05 bis etwa 1:0,55, umfasst.

12. Dentalmaterial nach einem der Anspruch 1 bis 10, **dadurch gekennzeichnet, dass** es ferner CaCl₂, ein Epoxid, Polycarboxylat und/oder ein fluoridhaltiges Material umfasst.

13. Verwendung des Dentalmaterials nach einem der Ansprüche 1 bis 12 zum Füllen oder Verschließen von Zahn- und/oder Zahnwurzelkavitäten und zum Abdecken von Pulpa und/oder Parodontium.

14. Verwendung nach Anspruch 13, **dadurch gekennzeichnet, dass** es sich bei Füllen von Zahnwurzelkavitäten um eine retrograde Zahnwurzelfüllung oder um eine anterograde Zahnwurzelfüllung/einzeitige definitive Wurzelkanalfüllung und bei dem Abdecken um eine Abdeckung von Perforationen handelt.

15. Verfahren zur Herstellung des Dentalmaterials nach einem der Ansprüche 1 bis 12, **dadurch gekennzeichnet, dass** Portlandzementklinker oder ein Portlandzement ähnlicher gipsfreier Zementklinker ein- oder mehrmals in einer oder in mehreren unterschiedlichen Mühlen gemahlen wird.
